# DEMANDE DE BREVET EUROPEEN

(11) **EP 0 556 075 A1**
(43) Date de publication de la demande: **18.08.1993**
(21) Numéro de dépôt: 93400101.7
(22) Date de dépôt: 18.01.1993
(51) Int. Cl.: A61M 1/16, A61J 1/00

(54) **Dispositif de préparation d'un produit pour dialyse et procédé mettant en oeuvre ce dispositif**

(30) Priorité: 13.02.1992 FR 9201850
(71) Demandeur: Lascombes, Jean-Jacques, F-31000 Toulouse (FR)
(72) Inventeur: Lascombes, Jean-Jacques, F-31000 Toulouse (FR)
(74) Mandataire: Morelle, Guy Georges Alain

(57) **Abrégé**

La présente invention concerne les dispositifs de préparation d'un produit pour dialyse.

Le dispositif selon l'invention qui comporte une enveloppe 1, une substance 2 contenue dans l'enveloppe, des premiers moyens 3 pour alimenter l'enveloppe en un fluide apte à dissoudre la substance, des seconds moyens 4 pour recevoir le soluté produit par la dissolution de la substance dans le fluide et le conduire à des moyens de traitement 5, ladite enveloppe présentant un axe de révolution 6 et comportant deux orifices 7, 8 de communication fluidique de même section réalisés dans sa paroi 9, chacun des orifices étant centré sur l'axe de révolution de l'enveloppe, à chacune de ses extrémités 53, 54, lesdits premiers et seconds moyens étant connectés respectivement aux deux dits orifices, se caractérise essentiellement par le fait que ladite enveloppe présente une structure symétrique de révolution de configuration cylindrique et qu'il comporte des moyens 40 pour maintenir l'enveloppe et la faire tourner autour d'un axe de rotation 46.

L'invention concerne également un procédé de préparation d'un produit pour dialyse mettant en oeuvre ce dispositif.

## Description

La présente invention concerne les dispositifs de préparation de produits pour dialyse, notamment les dispositifs qui permettent de préparer des produits pour dialyse à base de bicarbonate en poudre.

On connaît déjà des dispositifs qui permettent de réaliser des préparations de produits pour dialyse.

Ces dispositifs, notamment celui décrit dans le DE-U-91 11 524.8, comportent essentiellement une enveloppe contenant une substance, par exemple du bicarbonate en poudre, l'enveloppe comportant une entrée pour un liquide apte à dissoudre la substance et une sortie à laquelle est obtenue la solution. Ces dispositifs comportent en outre des moyens pour relier l'enveloppe à un système qui permet de faire la liaison avec le malade et de traiter le sang en vue d'effectuer sa dialyse.

Ces dispositifs selon l'Art Antérieur permettent de traiter les patients, mais les enveloppes qu'ils comportent présentent quelques inconvénients, provenant notamment du fait que leur structure en frome de tronc de cône provoque la formation d'amas de susbstance poudreuse adhérant aux parois de l'enveloppe, le liquide ayant ainsi tendance à ne pas se disperser dans la susbtance mais à former en quelque sorte "une cheminée" au sein de celle-ci. Il en résulte ainsi des pertes qui rendent l'utilisation de ces dispositifs d'un coût particulièrement élevé.

Or, l'importance de ces dispositifs pour la santé des êtres humains n'est plus à démontrer. Ils doivent donc être les moins onéreux possible pour pouvoir être accessibles à tous les malades et leur manipulation doit être la plus simple possible afin de rendre leur utilisation encore plus sécurisante.

La présente invention a ainsi pour but de réaliser un dispositif de préparation d'un produit pour dialyse, qui tente de répondre aux conditions mentionnées ci-dessus.

Plus précisément, la présente invention a pour objet un dispositif de préparation d'un produit pour dialyse, du type de celui décrit par le DEU-91 11 524.8, c'est-à-dire comportant une enveloppe, une substance de traitement contenue dans ladite enveloppe, des premiers moyens pour alimenter ladite enveloppe en un fluide apte à dissoudre ladite substance, des seconds moyens pour recevoir le soluté produit par la dissolution de ladite substance dans ledit fluide et le conduire à des moyens de traitement, ladite enveloppe présentant un axe de révolution et comportant deux orifices de communication fluidique de même section réalisés dans sa paroi, chacun des deux orifices étant centré sur l'axe de révolution de ladite enveloppe, à chacune de ses extrémités, lesdits premiers et seconds moyens étant connectés respectivement aux deux dits orifices, ledit dispositif étant caractérisé en ce que ladite enveloppe présente une structure symétrique de révolution de configuration cylindrique.

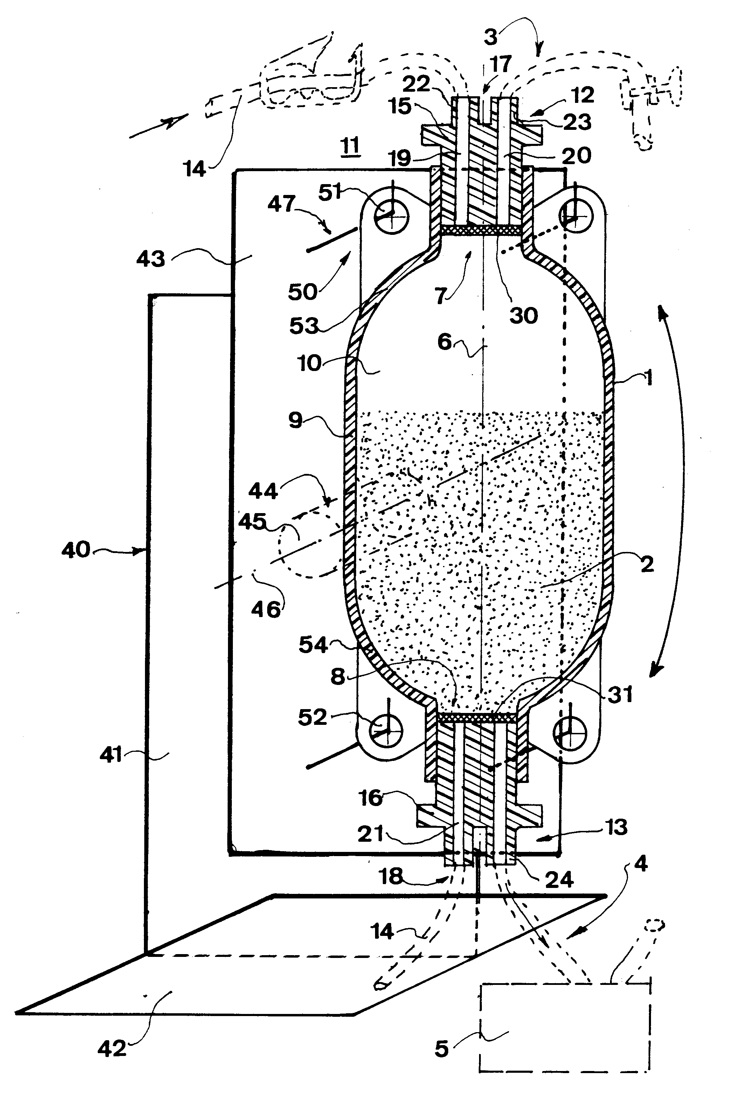

D'autres caractéristiques et avantages de la présente invention apparaîtront au cours de la description suivante donnée en regard du dessin annexé à titre illustratif mais nullement limitatif, dans lequel :

La Figure unique représente une vue en coupe d'un mode de réalisation avantageux d'un dispositif selon la présente invention pour la préparation d'un produit pour dialyse. La Figure unique représente un dispositif pour la préparation d'un produit pour dialyse. Ce dispositif comporte une enveloppe 1, une substance de traitement 2 contenue dans l'enveloppe, des premiers moyens 3 pour alimenter l'enveloppe en un fluide apte à dissoudre la substance, des seconds moyens 4 pour aspirer le soluté produit par la dissolution de la substance dans le fluide et le conduire à des moyens de traitement 5 qui n'ont été que très schématiquement illustrés sur la Figure car ils sont bien connus en eux-mêmes.

Selon une caractéristique importante de la présente invention, l'enveloppe 1 est de configuration cylindrique et est réalisée de façon à présenter un axe de révolution 6 et à comporter deux orifices de communication fluidique 7, 8 réalisés dans sa paroi 9 et de même section, permettant de mettre en relation l'intérieur 10 de l'enveloppe avec l'extérieur 11, chacun des orifices étant centré sur l'axe de révolution 6 de l'enveloppe, à chaque extrémité de celle-ci.

La structure telle que définie ci-dessus et qui se caractérisé par sa parfaite symétrie, rend très facile la fabrication de l'enveloppe et apporte au dispositif de nombreux avantages. Notamment, la forme cylindrique permet à l'enveloppe, qui est avantageusement réalisée dans un matériau comme du polyéthylène, d'accroître sa résistance à la pression et à la dépression.

Les premiers 3 et seconds moyens 4 comportent des tubulures fluidiques 14 et des moyens de connexion 12, 13 de ces tubulures fluidiques avec l'enveloppe 1. Ces tubulures fluidiques 14 permettent de véhiculer, soit le fluide qui permet de dissoudre la substance, soit le soluté vers le système de traitement 5 qui permet d'effectuer une dialyse selon une technique connue en elle-même.

Les moyens de connexion 12, 13 comportent des bouchons 15, 16, par exemple en polychlorure de vinyle, aptes à être enfoncés dans les orifices 7, 8, ces bouchons comportant en outre des moyens 17, 18 pour les relier aux tubulures fluidiques 14, ces moyens étant par exemple constitués par des percées 19, 20, 21, ... traversant les bouchons de part en part et par des connecteurs 22, 23, 24, ... des tubulures 14 avec les percées 19-21, ces derniers pouvant être constitués par des manchons solidaires des bouchons et sur lesquels ou dans lesquels peuvent être enfichées les tubulures fluidiques 14.

A ces bouchons sont avantageusement associés des filtres 30, 31 qui permettent d'obtenir, notamment en sortie de l'enveloppe 1, un soluté ne contenant pas de grains de poudre non dissous. Ces filtres sont par exemple réalisés en polycarbonate et constitués d'une trame dont les pores sont de l'ordre de 100 µ, ou même moins si cela est nécessaire. Chaque filtre est associé à un bouchon en étant par exemple positionné contre la face de ce bouchon qui se trouve tournée vers l'intérieur 10 de l'enveloppe 1 lorsque le bouchon est introduit dans un orifice. Avantageusement, ces faces de bouchon comportent un logement en creux dans le fond duquel est positionné le filtre qui se trouve ainsi protégé.

Le dispositif comporte en outre des moyens 40 pour maintenir l'enveloppe et la faire tourner autour d'un axe de rotation. Cette rotation doit être égale à sensiblement cent quatre vingts degrés. Ces moyens ont été très schématiquement illustrés sur la Figure unique.

Sans entrer dans les détails, ces moyens 40 comportent un support 41 apte à être positionné, par exemple par un pied 42, sur une surface de base, une embase 43, des moyens de soutien 47 de l'enveloppe sur l'embase 43 et des moyens 44 pour monter cette embase en rotation par rapport au support autour d'un arbre de rotation 45, de façon que l'axe 46 de cet arbre soit sensiblement horizontal quand la surface de base est horizontale.

Pour faciliter le montage de l'enveloppe sur l'embase de maintien 43, le dispositif comporte des moyens d'accrochage 50 de l'enveloppe sur l'embase. Ces moyens sont avantageusement constitués par au moins deux oeillets 51, 52 solidaires de l'enveloppe 1 et situés respectivement sensiblement aux deux extrémités 53, 54 de l'enveloppe. Avantageusement, ces oeillets peuvent être au nombre de quatre, comme illustré sur la Figure unique.

De cette façon, l'enveloppe peut être positionnée avec ses deux orifices 7, 8 sur un axe vertical et être retournée pour que l'orifice se trouvant dans la position basse se retrouve en position haute, et réciproquement pour l'autre orifice.

La substance 2 qui permet d'effectuer le traitement par dialyse est avantageusement constituée par du bicarbonate en poudre contenu dans l'enveloppe hermétiquement fermée quand elle est en attente d'utilisation.

Le dispositif décrit ci-dessus fonctionne de la façon suivante.

Lorsque l'on veut commencer un traitement par dialyse, on se procure une enveloppe 1 hermétiquement fermée et contenant une certaine quantité de substance de traitement, habituellement du bicarbonate en poudre dont les qualités, pour la réalisation d'une dialyse, sont bien connues en elles-mêmes et ne seront pas décrites ici. Le fluide utilisé pour la dissolution de la substance de traitement est alors de l'eau stérile et/ou pour hémodialyse.

L'enveloppe est positionnée sur son embase 43 et un bouchon 15, 16 est enfoncé dans chaque orifice 7, 8 de façon que la tubulure d'amenée de l'eau stérilisée soit connectée à l'orifice qui se trouve dans la position la plus basse. L'eau peut ainsi emplir l'enveloppe par le fond, en montant lentement et en la purgeant du gaz qu'elle contenait initialement ou qui peut se former lors de la dissolution de la poudre de bicarbonate. Il est bien évident que l'autre orifice est connecté à une tubulure de sortie apte à être reliée, de façon connue, aux moyens de traitement par dialyse 5.

Quand l'enceinte est complètement purgée, c'est-à-dire qu'elle ne contient plus de gaz, la tubulure de sortie est définitivement reliée aux moyens de traitement et, pour que ces moyens de traitement soient parfaitement alimentés en soluté à la bonne concentration en produit de traitement, l'enveloppe est pivotée autour de l'arbre de rotation 45. Dans cette position, le liquide de dissolution de la poudre arrive dans l'enveloppe, non pas par le bas, mais par le haut, et le soluté obtenu est aspiré par l'orifice qui se trouve alors dans le bas, dans un dosage adapté pour effectuer une dialyse conforme au traitement du sang selon les techniques classiques dans ce domaine. La Figure unique représente la position de l'enveloppe quand elle est en fonctionnement pour une dialyse.

Les percées 19-21, autres que celles qui sont utilisées pour le passage des tubulures d'amenée du liquide de dissolution et de sortie du soluté, sont soit bouchées soit connectées à d'autres tubulures pour assurer d'autres fonctions, par exemple des purges supplémentaires ou un amorçage de l'aspiration du soluté, et même, pour des dialyses importantes, la connexion en série de plusieurs enveloppes.

Notamment par la structure symétrique de révolution de l'enveloppe, le dispositif décrit ci-dessus répond bien aux conditions définies dans le préambule de la présente description.

## Revendications

1. Dispositif de préparation d'un produit pour dialyse, comportant une enveloppe (1), une substance (2) contenue dans ladite enveloppe, des premiers moyens (3) pour alimenter ladite enveloppe en un fluide apte à dissoudre ladite substance, des seconds moyens (4) pour recevoir le soluté produit par la dissolution de ladite substance dans ledit fluide et le conduire à des moyens de traitement (5), ladite enveloppe (1) présentant un axe de révolution (6) et comportant deux orifices (7, 8) de communication fluidique de même section réalisés dans sa paroi (9), chacun des orifices étant centré sur l'axe de révolution de ladite enveloppe, à chacune de ses extrémités (53, 54), lesdits premiers et seconds moyens étant connectés respectivement aux deux dits orifices, ***caractérisé en ce que*** ladite enveloppe présente une structure symétrique de révolution de configuration cylindrique.

2. Dispositif selon la Revendication 1, ***caractérisé en ce que*** lesdits premiers et seconds moyens (3, 4) comportent au moins une tubulure (14) et des moyens de connexion (12, 13) de ladite tubulure avec un orifice (7, 8).

3. Dispositif selon la Revendication 2, ***caractérisé en ce que*** les moyens de connexion (12, 13) comportent au moins un bouchon (15, 16), chaque bouchon comportant des moyens (17, 18) pour le relier à une tubulure (14).

4. Dispositif selon la Revendication 3, ***caractérisé en ce qu***'au moins un filtre (30, 31) est associé à un bouchon.

5. Dispositif selon la Revendication 4, ***caractérisé en ce que*** ledit filtre est associé au bouchon en étant disposé dans le fond d'un logement réalisé sur la face du bouchon tournée vers l'intérieur de ladite enveloppe.

6. Dispositif selon l'une des Revendications 3 à 5, ***caractérisé en ce que*** lesdits moyens (17, 18) pour relier un bouchon (15, 16) à une tubulure (14) comportent au moins une percée (19-21) traversant de part en part ledit bouchon.

7. Dispositif selon l'une des Revendications précédentes, ***caractérisé en ce qu***'il comporte des moyens (40) pour maintenir l'enveloppe et la faire tourner autour d'un axe de rotation (46).

8. Dispositif selon la Revendication 7, ***caractérisé en ce que*** les moyens de maintien (40) comportent un support (41) apte à être positionné sur une surface de base, une embase (43), des moyens (47) de soutien de ladite enveloppe sur ladite embase et des moyens (44) pour monter cette embase en rotation par rapport au support autour d'un arbre de rotation (45), de façon que l'axe (46) de cet arbre soit sensiblement horizontal quand la surface de base est horizontale.

9. Dispositif selon la Revendication 8, ***caractérisé en ce que*** les moyens de soutien sur ladite embase (43) de ladite enveloppe comportent des moyens d'accrochage (50) solidaires de ladite enveloppe.

10. Dispositif selon la Revendication 9, ***caractérisé en ce que*** les moyens d'accrochage (50) sont constitués par au moins deux oeillets (51, 52) solidaires de l'enveloppe (1), ces deux oeillets étant situés respectivement sensiblement aux deux extrémités (53, 54) de ladite enveloppe.

11. Dispositif selon l'une des Revendications précédentes, ***caractérisé en ce que*** ladite substance (2) est constituée par du bicarbonate en poudre.

12. Procédé de préparation d'un produit pour dialyse mettant en oeuvre le dispositif selon les Revendications 1 et 7, ***caractérisé en ce qu***'il consiste :
- dans un premier temps, à connecter une tubulure d'amenée d'eau stérile à l'orifice de l'enveloppe qui se trouve dans la position la plus basse et à remplir ainsi ladite enveloppe par le fond, l'orifice de l'enveloppe qui se trouve dans la position la plus haute étant connecté à une tubulure de sortie reliée à des moyens de traitement ;
- dans un second temps, à faire pivoter l'enveloppe de cent quatre vingt degrés de manière à introduire l'eau stérile dans l'enveloppe par le haut, le soluté produit étant alors aspiré par l'orifice qui se trouve dans la position la plus basse.
